# FASCICULE DE BREVET EUROPEEN

(11) **EP 2 424 837 B1**
(45) Date de publication et mention de la délivrance du brevet: **27.04.2016**
(21) Numéro de dépôt: 10715257.1
(22) Date de dépôt: 27.04.2010
(51) Int. Cl.: C07D 201/08, C07D 223/10

(54) **PROCEDE DE PREPARATION DE LACTAMES**
VERFAHREN ZUR HERSTELLUNG VON LACTAMEN
PROCESS FOR THE PREPARATION OF LACTAMS

(30) Priorité: 27.04.2009 FR 0952744
(43) Date de publication de la demande: 07.03.2012
(73) Titulaire: Rhodia Opérations, 93306 Aubervilliers (FR)
(72) Inventeur: LECONTE, Philippe, F-68150 Ribeauvillé (FR)
(74) Mandataire: Vande Gucht, Anne
(86) Numéro de dépôt international: PCT/EP2010/055588
(87) Numéro de publication internationale: WO 2010/125040

(56) Documents cités:
- US-A- 2 357 484
- US-B1- 7 060 820
- US-B1- 7 060 820

## Description

La présente invention concerne un procédé de préparation de lactames par hydrolyse cyclisante d'aminonitrile correspondant.

Les lactames aliphatiques, tels que notamment l' ε-caprolactame sont des composés de base pour la préparation de polymères tels que les polyamides. L' ε-caprolactame est le monomère de base pour la fabrication du polyamide 6, un polymère utilisé dans de nombreuses applications telles que, par exemple, l'industrie du textile, l'industrie des pièces moulées ou injectées pour de nombreux domaines tels que l'automobile, les connecteurs électriques, les appareils électroménagers, etc...

Parmi les procédés connus de fabrication d'un lactame, le procédé concerné par l'invention consiste à faire réagir un aminonitrile avec de l'eau en présence d'un catalyseur pour réaliser une hydrolyse cyclisante de l'aminonitrile en lactame.

Ce procédé est décrit dans de nombreux brevets. On peut citer à titre d'exemple le brevet US 2 357 484 qui décrit un procédé de préparation en phase vapeur de lactame, consistant à faire passer un mélange d'eau et d'aminonitrile sur un catalyseur, tel que l'alumine activée, un gel de silice ou de l'acide borophosphorique.

Le brevet US 4 628 085 propose également un procédé de préparation de lactames consistant à mettre en contact, en phase vapeur, un aminonitrile aliphatique ou aromatique et de l'eau en présence d'un catalyseur à base de silice sous forme sphérique ayant une surface BET supérieure à 350 m²/g et un diamètre de pore inférieur à 20 nm, généralement en présence d'ammoniac et d'hydrogène.

Il a également été proposé par le brevet EP 0805801, un procédé de fabrication de lactame par hydrolyse cyclisante d'un aminonitrile en présence d'un catalyseur, une alumine activée présentant des caractéristiques particulières de porosité et surface spécifique. Ce catalyseur présente une durée de vie très importante par rapport aux autres catalyseurs. D'autres caractéristiques de ce procédé ont été décrites dans les brevets EP0659741, EP0805801, EP1089972, EP1098875, EP1370524. Le brevet US7060820 divulgue un procédé de préparation en phase vapeur de caprolactame comprenant une succession de zones de réaction contenant un catalyseur entrecoupées par des zones de refroidissement.

Cette réaction est généralement réalisée dans un réacteur tubulaire, le catalyseur étant disposé dans chaque tube.

La réaction d'hydrolyse cyclisante est exothermique et donc il est nécessaire d'évacuer les calories dégagées pour maintenir une température qui permette d'une part d'avoir une vitesse de réaction suffisante et d'autre part, de minimiser la formation de sous-produits.

Un des buts de la présente invention est de proposer un procédé permettant de contrôler la température dans le réacteur tubulaire pour avoir une productivité élevée tout en conservant une sélectivité en lactame très élevée.

A cet effet, l'invention propose un procédé de fabrication de lactame par réaction d'un aminonitrile avec de l'eau en présence d'un catalyseur consistant à mettre en contact en phase vapeur, de l'eau et l'aminonitrile, à faire passer le mélange de vapeurs à travers un lit de catalyseur disposé dans au moins un tube formant un réacteur et à récupérer le lactame en sortie du tube.

Selon l'invention, le procédé comprend des moyens de refroidissement pour permettre de maintenir la température, en tout point du tube, à une valeur inférieure à 340 °C et à une valeur supérieure à 280°C.

En effet, pour éviter la formation des sous-produits et donc augmenter la sélectivité de la réaction en caprolactame, il est important de maintenir la température dans le tube réacteur à une valeur inférieure à 340°C, notamment dans la première partie amont du tube là où la quantité de chaleur dégagée par la réaction est la plus importante.

En outre, pour obtenir une productivité optimale du procédé, il est également nécessaire que la température dans le tube réacteur ne devienne pas inférieure à 280°C environ.

Selon l'invention, ce contrôle de la température dans le tube du réacteur et notamment sur toute la longueur du tube est obtenu par l'utilisation d'au moins deux moyens différents de refroidissement :
- Un premier moyen de refroidissement d'une première partie du réacteur appelée «partie amont » présentant un pouvoir d'évacuation des calories suffisant pour éviter une élévation de la température à une valeur supérieure à 340°C environ.
- Un second moyen de refroidissement de l'autre partie du tube réacteur appelée "partie aval" permettant de maintenir la température du tube à une valeur supérieure à 280°C environ et inférieure à 340°C environ. Le second moyen de refroidissement présente un pouvoir d'évacuation des calories différent de celui du premier moyen. En effet, la quantité de calories dégagées par la réaction entre l'eau et l'aminonitrile est plus faible dans cette partie du tube, compte tenu que les concentrations en eau et aminonitrile ont diminué. De ce fait, le risque d'avoir des températures très élevées est diminué. Toutefois, il est nécessaire de maintenir la température à un certain niveau pour avoir une vitesse de réaction et productivité suffisantes.

Dans un mode de réalisation préféré de l'invention, les moyens de refroidissement sont constitués par des dispositifs permettant la circulation d'un fluide de refroidissement tel que des huiles et/ou des sels fondus. La température de ce fluide de refroidissement est différente dans les deux zones de refroidissement.

Avantageusement la température du premier moyen de refroidissement est comprise entre 280 et 310°C. De préférence la température du deuxième moyen de refroidissement est comprise entre 290 et 340°C.

En outre, sans sortir du cadre de l'invention, il est possible d'utiliser un nombre de zones de refroidissement supérieur à 2, chaque moyen de refroidissement permettant de contrôler une partie du tube réacteur, et la capacité d'évacuation des calories de chaque moyen étant adaptée à la quantité de chaleur dégagée par la réaction dans la partie du tube concernée, par adaptation de la température.

De manière générale, le réacteur utilisé est un réacteur tubulaire comprenant un nombre élevé de tubes disposés parallèlement. Chaque tube est rempli avec un catalyseur solide, avantageusement en poudre ou en granulés. L'extrémité amont de ce réacteur comprend des moyens d'alimentation de l'aminonitrile et de l'eau sous forme vapeur.

Les moyens de refroidissement sont généralement constitués par la circulation d'un fluide caloporteur liquide baignant les tubes du réacteur.

Les moyens de refroidissement peuvent être uniquement constitués par une entrée d'un fluide caloporteur dans la calandre du réacteur et une évacuation du dit fluide. Les tubes sont ainsi immergés dans le fluide caloporteur.

Ainsi, dans un mode de réalisation de l'invention, le réacteur tubulaire comprend une zone amont, dans laquelle circule un fluide caloporteur autour des tubes, et une seconde zone aval dans laquelle circule un fluide caloporteur généralement à une température supérieure à celle du caloporteur circulant dans la première. Chaque zone de refroidissement peut-être séparée avantageusement par une plaque d'étanchéité.

La partie amont du réacteur ou du tube représente, selon une caractéristique de l'invention, de 0,1 à 0,3 fois la longueur du réacteur ou du tube. Ainsi, pour un réacteur à l'échelle industrielle, la longueur de la partie amont est comprise entre 0,5 m et 1,5 m, la longueur totale du tube ou réacteur étant avantageusement comprise entre 3,5 m et 7 m.

Le procédé d'hydrolyse d'un aminonitrile tel que l'aminocapronitrile en caprolactame est réalisée avec un catalyseur d'hydrolyse, notamment une alumine activée éventuellement dopée. Les catalyseurs convenables pour l'invention sont décrits, par exemple, dans les brevets européens n° 0805801 et n° 1098875. D'autres catalyseurs d'hydrolyse solides peuvent être utilisés comme ceux décrits dans le brevet EP 0659741.

L'aminonitrile utilisé dans le procédé de l'invention est plus particulièrement un aminonitrile aliphatique linéaire ou ramifié ayant de 3 à 12 atomes de carbone.

A titre d'exemples, on peut citer les aminonitriles obtenus par hydrogénation d'une fonction nitrile de composés dinitriles tels que l'adiponitrile, le méthylglutaronitrile, l'éthylsuccinonitrile, le diméthylsuccinonitrile, le malononitrile, le succinonitrile, le glutaronitrile et le dodécane dinitrile.

L'aminonitrile le plus important est l'aminocapronitrile obtenu par hydrogénation partielle de l'adiponitrile. L'hydrolyse cyclisante de ce composé conduit à l'ε-caprolactame qui est un monomère important pour la fabrication des polyamides, notamment du polyamide 6.

L'aminonitrile et l'eau sont introduits dans les tubes du réacteur sous forme vapeur.

Avantageusement, l'aminonitrile est vaporisé par le contact avec la vapeur d'eau et donc la durée de contact entre la vapeur et l'aminonitrile en absence de catalyseur est minimisée. Ce modèle de réalisation d'introduction des réactifs est décrit dans le brevet européen n° 1089972.

D'autres caractéristiques du procédé d'hydrolyse d'aminonitrile en lactame sont décrits dans la littérature comme, par exemple, dans le brevet EP 0938473. Le lactame récupéré est purifié par distillation pour notamment séparer l'eau et les produits légers et lourds.

Des procédés de purification des lactames sont décrits dans les brevets européens n° 0922027, 1105374, 1131287, 1242375.

Ces procédés de purification comprennent des étapes de traitement acide sur résine échangeuse d'ions ainsi que des colonnes de déshydratation pour éliminer l'eau du lactame. L'eau ainsi récupérée dans la colonne de déshydratation peut être avantageusement utilisée comme réactif dans l'étape d'hydrolyse cyclisante. La vaporisation de ce flux aqueux peut être avantageusement obtenue par échange de chaleur avec les vapeurs sortant du réacteur d'hydrolyse pour obtenir une pré-condensation de celles-ci.

L'aminonitrile utilisé dans le procédé de l'invention peut être obtenu par tout procédé connu. Toutefois, dans un mode de réalisation préféré de l'invention, notamment quand le lactame est l'ε-caprolactame, l'aminonitrile est l'aminocapronitrile obtenu par hémihydrogénation de l'adiponitrile. Ces procédés d'hémihydrogénation sont décrits dans de nombreux brevets tels que EP 1397346, EP 1397345, EP 0797568, EP 0925106, EP 1127047.

Les produits d'hémihydrogénation comprenant le solvant, la diamine, l'aminonitrile et le dinitrile non hydrogéné sont séparés par distillations successives. Des procédés de séparation des produits issus de l'hémihydrogénation sont décrits dans la littérature et notamment les brevets européens n° 1071657, n° 1077932 et le brevet WO2007/045750.

Dans le cas où le solvant de la réaction d'hémihydrogénation est l'eau, celle-ci après séparation par distillation peut être avantageusement utilisée dans une autre étape du procédé de préparation du caprolactame tel que, par exemple, dans l'étape de pré-lavage avant régénération des résines échangeuses d'ion utilisées dans la purification du caprolactame.

Dans le cas de l'hémihydrogénation de l'adiponitrile, l'hexaméthylène diamine est séparée de l'aminocapronitrile par distillation dans une colonne à distiller (A) comme fraction de tête de la colonne. Cette hexaméthylène diamine brute peut avantageusement subir une autre distillation dans une colonne à distiller (B). L'hexaméthylène diamine pure est alors soutirée en pasteurisé sur un des plateaux de la colonne (B) tandis qu'en tête de colonne, les composés dits légers sont éliminés, et la fraction de pied ou culot étant recyclé comme reflux dans la colonne à distiller (A).

D'autres détails, avantages de l'invention apparaissent au vu des exemples donnés ci-dessous à titre indicatif et illustratif uniquement. Les exemples ci-dessous sont réalisés dans un réacteur multitubulaire vertical de 55 tubes, de 4 m de longueur. Chaque tube est rempli par une alumine activée utilisée comme catalyseur et présentant les caractéristiques suivantes :
- Surface spécifique (SS) : 139 m²/g
- Volume poreux total : 117ml/100g
- Volume poreux correspondant aux pores de diamètre supérieur à 500 Å : 50ml/100g
- Volume poreux correspondant aux pores de diamètre supérieur à 200 Å : 70ml/100g
- Volume poreux correspondant aux pores de diamètre supérieur à 70 Å : 116ml/100g.

Deux des tubes remplis contiennent en outre une sonde de température pour suivre le profil de température. Pour garantir une bonne homogénéité de l'échange thermique sur chacun des tubes, des chicanes et des pompes de recirculation sont disposés de manière optimisée en fonction des connaissances générales dans le domaine des échanges thermiques. Ainsi la différence maximale de température du fluide caloporteur entre son entrée dans la calandre du réacteur et sa sortie est de 2°C.

### Exemple 1 comparatif :

Le réacteur ne possède qu'une seule zone de refroidissement ou un seul moyen de refroidissement. Le caloporteur entre en bas de la calandre du réacteur et ressort en haut.

Un flux vapeur composé de 38 kg/h d'aminocapronitrile (ACN) et de 24,5 kg/h d'eau alimente en continue le haut du réacteur d'hydrolyse.

La température d'entrée du caloporteur est fixée pour maintenir la température au point le plus chaud du catalyseur au maximum à 340 ± 1°C. Ce point chaud se situe entre 0,2 et 0,3 m en dessous de l'entrée des tubes du réacteur contenant le catalyseur.

Le tableau ci-dessous donne la température d'entrée du caloporteur, la conversion de l'ACN et la sélectivité en caprolactame (CL) en fonction du temps.

| | | | |
|---|---|---|---|
| Durée en heures | 24 | 300 | 500 |
| Température Caloporteur °C | 282 | 286 | 289 |
| Conversion ACN % | 99,4 | 99,2 | 99,1 |
| Sélectivité CL % | ≥99,8 | ≥99,8 | ≥99,8 |

Afin de compenser la désactivation du catalyseur et maintenir le point chaud à 340°C, la température du caloporteur doit être progressivement augmentée.

Malgré l'augmentation de température, la sélectivité en caprolactame n'est pas affectée mais la conversion en ACN diminue progressivement.

### Exemple 2

L'exemple 1 est reconduit de manière similaire mais avec la différence que le réacteur dispose de deux zones de refroidissement distinctes séparées par une plaque. Ces zones sont régulées indépendamment en température.

La première zone d'une longueur de 1 m est utilisée pour limiter le point chaud à l'entrée du réacteur au maximum à 340°C.

La deuxième zone de 3 m autorise un taux de conversion de l'ACN le plus élevé possible.

Le tableau ci-dessous donne les températures d'entrée du caloporteur dans les deux zones, la conversion de l'ACN et la sélectivité en caprolactame (CL) en fonction du temps.

| | | | |
|---|---|---|---|
| Durée en heures | 24 | 300 | 500 |
| Température Caloporteur °C Zone 1 (amont) | 282 | 286 | 289 |
| Température Caloporteur °C Zone 2 (aval) | 300 | 300 | 300 |
| Conversion ACN % | 99,5 | 99,5 | 99,5 |
| Sélectivité CL % | ≥99,8 | ≥99,8 | ≥99,8 |

On constate après 500 heures de fonctionnement continu que la conversion de l'ACN est maintenue en fixant la température de la seconde zone à 300°C.

## Revendications

1. Procédé de fabrication de lactame par réaction d'un aminonitrile avec de l'eau consistant à mettre en contact en phase vapeur de l'eau et de l'aminonitrile, à faire passer le mélange de vapeurs à travers un lit de catalyseur disposé dans au moins un tube formant un réacteur et à récupérer le lactame en sortie de tube, **caractérisé en ce que** la température dans le tube est contrôlée par des moyens de refroidissement pour maintenir la température dans toute la longueur du tube à une valeur comprise entre 280° et 340°C, les dits dispositifs de refroidissement comprenant au moins deux moyens distincts dont l'un est destiné à contrôler la température dans la partie amont du tube pour maintenir la température à une valeur inférieure à 340°C, et le deuxième étant disposé sur l'autre partie aval du tube pour maintenir la température du tube à une valeur supérieure à 280°C et inférieure à 340°C.

2. Procédé selon la revendication 1, **caractérisé en ce que** la température du moyen destiné à contrôler la température dans la partie amont du tube est comprise entre 280 et 310°C.

3. Procédé selon la revendication 1 ou 2, **caractérisé en ce que** la température du moyen disposé sur la partie aval du tube est comprise entre 290 et 340°C.

4. Procédé selon l'une des revendications précédentes, **caractérisée en ce que** la partie amont du tube a une longueur comprise entre 0,5 m et 1,5 m, la longueur totale du tube étant comprise entre 3,5 m et 7 m.

5. Procédé selon l'une des revendications précédentes, **caractérisé en ce que** l'aminonitrile est l'aminocapronitrile, le lactame étant l'ε-caprolactame.

## Patentansprüche

1. Verfahren zur Herstellung von Lactamen durch Umsetzung eines Aminonitrils mit Wasser, das darin besteht, dass man Wasser und Aminonitril in der Dampfphase in Berührung bringt, die Mischung von Dämpfen über eine in mindestens einem einen Reaktor bildenden Rohr angeordnete Katalysatorschüttung leitet und das Lactam am Ausgang des Rohrs gewinnt, **dadurch gekennzeichnet, dass** die Temperatur in dem Rohr durch Kühlmittel gesteuert wird, um die Temperatur über die gesamte Länge des Rohrs bei einem Wert zwischen 280 und 340°C zu halten, wobei die Kühlvorrichtungen mindestens zwei verschiedene Mittel umfassen, von denen das eine zur Steuerung der Temperatur im stromaufwärts gelegenen Teil des Rohrs bestimmt ist, um die Temperatur auf einem Wert unter 340°C zu halten, und das zweite an dem anderen stromabwärts gelegenen Teil des Rohrs angeordnet ist, um die Temperatur des Rohrs bei einem Wert von mehr als 280°C und weniger als 340°C zu halten.

2. Verfahren nach Anspruch 1, **dadurch gekennzeichnet, dass** die Temperatur des zur Steuerung der Temperatur im stromaufwärts gelegenen Teil des Rohrs bestimmten Mittels zwischen 280 und 310°C liegt.

3. Verfahren nach Anspruch 1 oder 2, **dadurch gekennzeichnet, dass** die Temperatur des auf dem stromabwärts gelegenen Teil des Rohrs angeordneten Mittels zwischen 290 und 340°C liegt.

4. Verfahren nach einem der vorhergehenden Ansprüche, **dadurch gekennzeichnet, dass** der stromaufwärts gelegene Teil des Rohrs eine Länge zwischen 0,5 m und 1,5 m aufweist, wobei die Gesamtlänge des Rohrs zwischen 3,5 m und 7 m liegt.

5. Verfahren nach einem der vorhergehenden Ansprüche, **dadurch gekennzeichnet, dass** es sich bei dem Aminonitril um Aminocapronitril handelt, wobei es sich bei dem Lactam um ε-Caprolactam handelt.

## Claims

1. Process for manufacturing lactam by reaction of an aminonitrile with water that consists in bringing into contact, in the vapour phase, water and aminonitrile, in passing the mixture of vapours through a catalyst bed positioned in at least one tube that forms a reactor and in recovering the lactam at the tube outlet, **characterized in that** the temperature in the tube is controlled by cooling means in order to maintain the temperature throughout the length of the tube at a value between 280° and 340°C, said cooling devices comprising at least two distinct means, one of which is intended to control the temperature in the upstream part of the tube in order to maintain the temperature at a value below 340°C, and the second being positioned around the other downstream part of the tube in order to maintain the temperature of the tube at a value above 280°C and below 340°C.

2. Process according to Claim 1, **characterized in that** the temperature of the means intended to control the temperature in the upstream part of the tube is between 280°C and 310°C.

3. Process according to Claim 1 or 2, **characterized in that** the temperature of the means positioned around the downstream part of the tube is between 290°C and 340°C.

4. Process according to one of the preceding claims, **characterized in that** the upstream part of the tube has a length between 0.5 m and 1.5 m, the total length of the tube being between 3.5 m and 7 m.

5. Process according to one of the preceding claims, **characterized in that** the aminonitrile is aminocapronitrile, the lactam being ε-caprolactam.
